# EUROPEAN PATENT APPLICATION

(11) **EP 0 522 484 A1**
(43) Date of publication of application: **13.01.1993**
(21) Application number: 92111424.5
(22) Date of filing: 06.07.1992
(51) Int. Cl.: A61F 5/01

(54) **Orthopedic appliance with improved adjustability**

(30) Priority: 10.07.1991 IT PN910021 U
(71) Applicant: TECHNOFIN S.r.l., I-00155 Roma (IT)
(72) Inventor: Zancai, Lucio, I-33084 Cordenons, Pordenone (IT); Kos, Edo, I-33080 Fiume Veneto, Pordenone (IT)
(74) Representative: Grünecker, Kinkeldey, Stockmair & Schwanhäusser Anwaltssozietät

(57) **Abstract**

Appliance for the adjustment and control of the free rotation of two arms (1, 2) about a pivot pin (3), for particular use in the field of orthopedics (trainers) for the control of the minimum and maximum rotation of two limbs interconnected by an articulation, and adapted to permit the continuous adjustment of the angle of rotation without the necessity of dismantling the appliance.

The appliance includes an adjustment bar housing at least one spring, preferably a tension spring, and at least one threaded member the position of which is adjustable by the action thereon of a rotatable internally threaded knurled bushing, and at least one flexible cable having one of its ends secured to a pin mounted adjacent the pivot pin of the two arms, and its other end to a stop member cooperating with said spring.

## Description

The invention relates to an apparatus for controlling the rotatioon of two hinged arms relative to one another, for particular use in the field of orthopedics as an appliance for controlling the articulation and ligaments of a diseased or injured limb.

As generally known, orthopedic devices or appliances also known as "trainers" are universally used during rehabilitation of injured articulations or in any other case necessitating the movements of such articulations to be controlled and/or restricted between two definite limits in a manner permitting the movements of the affected limbs to take place solely as a rotation relative to one another about the common articulation and in a substantially fixed plane.

Known appliances of this type may be of any of several constructions which for the sake of brevity shall only be described as far as required for the understanding of the present invention.

Suffice it to recall that these appliances comprise two movable elements of substantially flat shape and a length which may vary in accordance with requirements, and rotatable relative to one another about a common pivot so as to be substantially retained in a single plane irrespective of their position relative to the common pivot, these elements being devised to offer considerable resistance to any deformation relative to the pivot and to any deflection out of their common plane.

An appliance of this type is intended to be secured to the affected limb in alignment of its pivot with the axis of the respective articulation, the two flat elements being secured to the limb on opposite sides of the articulation by means of neoprene straps or similar devices, so that the two parts of the limb are capable of free rotation relative to one another about a pre-set angle strictly in the fixed plane.

Although in theory these appliances are highly effective, their practical use is hampered by shortcomings of two different kinds resulting from their being provided with a number of dismountable components.

A first shortcoming is due to the fact that in a trainer of this type, the mechanism of which is well known to those skilled in the art and must therefore not be explained, the two flat elements or arms connected to the common pivot are capable of being rotated relative to one another only between a discrete number of pre-fixed positions, so that it is not possible to adjust their relative rotation to any freely determinable angle.

It now frequently happens that a trainer of this type is called upon to control the movements of a limb at a highly accurate setting, in which case the step-wise setting feature of conventional appliances is found inadequate.

A second shortcoming springs directly from the construction of the mechanism employed. The setting of the angles of rotation is in fact obtained with a system of coaxial bores in a central structure, and associated stop pins employed for arresting the inner ends of the rotatable arms, the overall structure being closed by a cover acting to prevent the stop pins from dropping out. In this case, an alteration of the angle of free rotation of the two arms requires the trainer to be removed from the affected limb, the cover to be removed from the central structure, and the stop pins to be shifted to another pair of aligned bores.

At the end of this adjustment operation, the cover has to be replaced on the central structure, whereupon the trainer is again applied to the affected limb. Although these operations are relatively simple, there is the real danger of the stop pins getting lost, and above all, of a faulty setting requiring the entire operation to be repeated.

It would therefore be desirable, and is in fact an object of the present invention, to provide a trainer appliance in which the shortcomings discussed above are radically eliminated, which is readily and quickly adjustable and of reliable and economical construction.

These and other objects are attained according to the invention by the employ of components having particular characteristics as will be explained in the following description.

The invention will be more fully understood from the following description, given by way of example with reference to the accompanying drawings, wherein:
fig. 1 shows a top plan view of a first embodiment of the trainer appliance according to the invention with its arms in the fully opened position,
fig. 2 shows a top plan view of the trainer appliance of fig. 1 with its arms in a steeply angled position,
fig. 3 shows an enlarged detail from fig. 1,
fig. 4 shows a modified embodiment of the invention, and

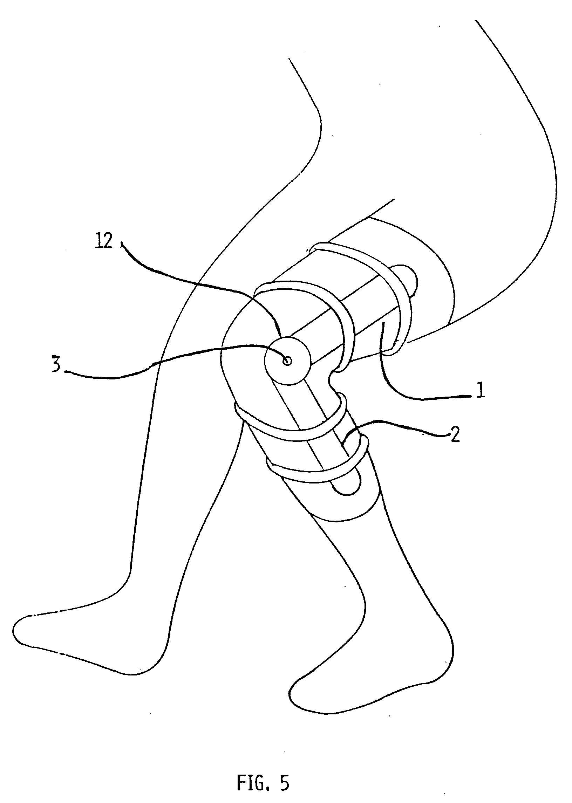

fig. 5 shows an example of the employ of the trainer appliance according to the invention.

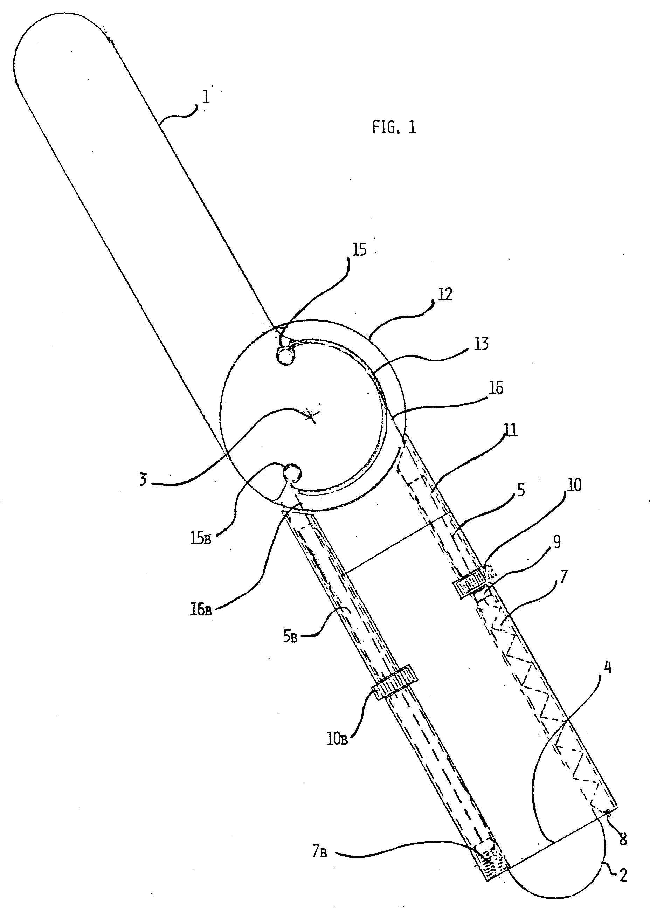

With reference to fig. 1, the appliance according to the invention is composed of two arms 1 and 2 mounted on a connecting pivot pin 3 for rotation relative to one another

Mounted on one of the two arms, e.g. on arm 2, is a flat bar 4 having dimensions corresponding to those of the arm and provided with two extensions 5 and 5b extending along its two lateral sides in a symmetric arrangement.

The two extensions 5 and 5b are hollow over their full lengths, the hollow interior of each extension housing (with reference to extension 5) a tension spring 7 having one of its ends secured to the outer end 8 of the respective extension, and its other end to a substantially cylindrical plug 9 contained in the hollow extension.

Connected to the other end face of plug 9 is one end of a flexible cable to be referred to in detail as the description proceeds.

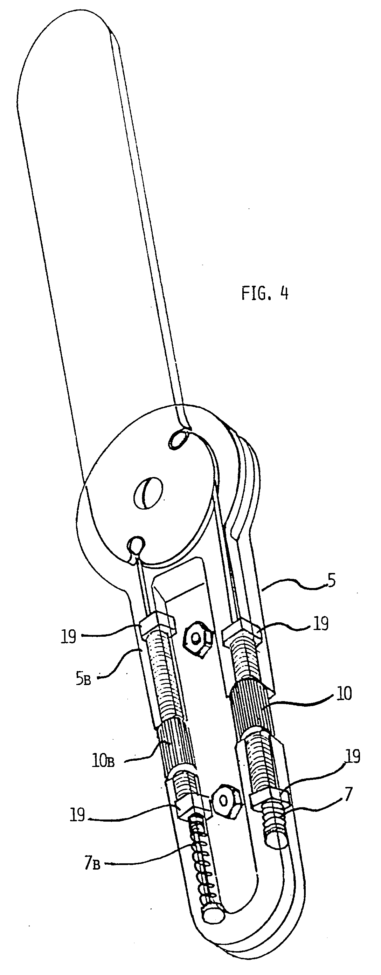

Provided at a fixed intermediate position of extension 5 is a knurled bushing 10 mounted for rotation about the hollow interior of the extension and, as shown in fig. 4, formed with internal threads permanently engaged with outer threads of an elongate threaded member 11 housed in the bore of extension 5 in a portion thereof opposite to spring 7 with respect to plug 9.

Threaded member 11 is hollow, and plug 9 has a diameter preventing it from passing through the hollow interior of threaded member 11.

The trainer appliance has a central body 12 fixedly connected to the adjustment arm 2 and formed with an arcuate groove 13 preferably coaxial with pivot pin 3 and extending over an arc of about 200^{o}.

Disposed adjacent the two ends of groove 13 are two pins 15 and 15b to which the free ends of the two flexible cables 16 and 16B, preferably steel cables, are secured.

The two cables are guided within groove 13 over a certain length and extend from there through the bore of the respective threaded member 11 to have their other ends secured to the associated plugs 9. The hollow extension 5 thus houses, in sequence from central pivot pin 3, the threaded member 11, internally threaded bushing 10, plug 9, and tension spring 7.

As already explained, flexible cable 16 extends from its anchoring point on plug 9 through the bore of threaded member 11 and is guided over a certain length in arcuate groove 13, to have its other end connected to pin 15.

The described construction is identically repeated with regard to the other extension 5b with components corresponding to the ones described being designated by the same reference numerals amended with the suffix b. This construction needs therefore not be described again.

The function of the described appliance will now be obvious to one skilled in the art. The angle of free rotation of the two arms 1 and 2 relative to one another can be determined between end positions by suitably adjusting threaded members 11, 11b by rotating knurled bushings 10, 10b.

The rotation of knurled bushing 10 in fact causes threaded member 11 to be displaced within the bore of extension 5, and since the internal diameter of threaded member 11 is sufficiently small, plug 9 is unable to pass thereinto, but may at the most abut the end face of the member, from which it becomes evident that the maximum rotary adjustment of knurled bushing 10 determines the maximum counterclockwise rotation angle of arm 1 relative to arm 2 as shown in fig. 1.

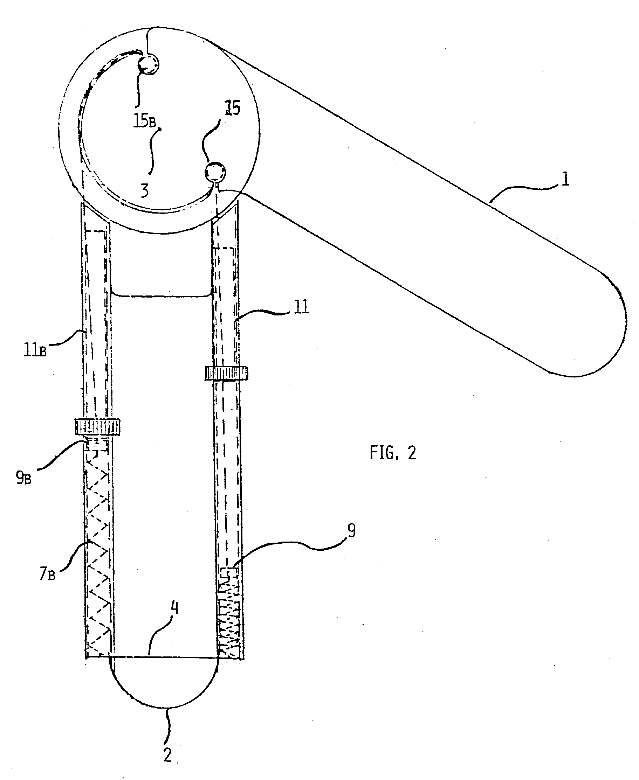

The maximum clockwise rotation angle of arm 1 relative to arm 2 as shown in fig. 2 may then of course be determined by the adjustment of knurled bushing 10b acting on an adjustment mechanism associated to extension 5b and functioning in the same manner as described.

At this point it is evident that the two end positions of arms 1 and 2, and thus their angle of free rotation relative to one another, can be readily determined by suitably acting on the two knurled bushings 10 and 10b. The end positions of arms 1 and 2 may then be fixedly set by locking bushings 10 and 10b against further rotation; this can be readily accomplished by any of several known means.

The two tension spring 7 and 7b are provided for constantly maintaining the two flexible cables 11 and 11b under tension.

The construction used in the embodiment of figs. 1 and 2 for determining both end positions of arms 1 and 2 may of course also be used for determining only one of these end positions, in which case the other end position may be determined in a fixed manner as by means of a fixed abutment stop between the arms acting to prevent any rotation thereof relative to one another beyond a determined maximum or minimum angle.

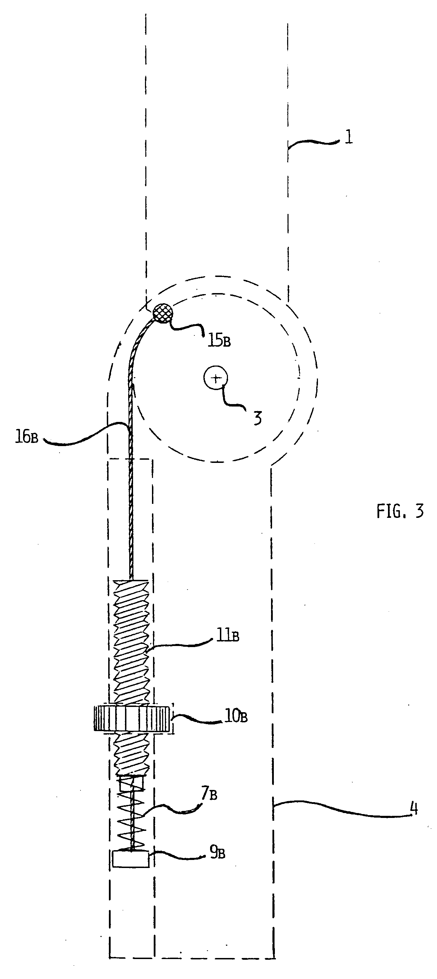

In this case the adjustment mechanism according to the invention is of course only provided on one side of bar 4 as shown in fig. 3, with all of the considerations set forth above remaining valid.

With reference to fig. 3, there is additionally shown a useful modification of the described mechanism: according to this modification, the spring 7b provided for maintaining cable 16b under constant tension is not mounted between plug 9b and the end 8 of the respective bar extension, but is rather interposed between the outer end of threaded member 11b and plug 9b. It is obvious that in this case spring 7b is a compression spring rather than a tension spring, and that the anchoring point at 8 needs not be provided.

A further improvement of the construction described may be obtained by using a configuration of the type shown in fig. 4. In this embodiment, the flat bar 4 supporting the adjustment mechanisms has been eliminated, these mechanisms being directly housed in one of the two arms suitably dimensioned for this purpose.

With reference to fig. 4, it is noted that use is made of compression springs 7 and 7b as in the modification of fig. 3, and that arm 1 itself functions as the adjustment element to which purpose its opposite sides are formed with hollow sections for housing all of the functional components described above.

In the present case it is noted that the hollow interior of each lateral section 5, 5b is of square cross-sectional shape, and that the ends of threaded members 11 and 11b are provided with square-section blocks 19, 19b fixedly secured thereto for preventing the threaded members from following the rotation of threaded bushings 10, 10b during an adjustment operation.

This embodiment offers the advantages of a simplified construction and a more facile maintenance of the appliance.

It is now evident that the present invention eliminates the shortcomings of hitherto employed trainer appliances, since the rotatable threaded bushings 10, 10b permit adjustments to be carried out in a completely continuous fashion and with a high degree of precision without requiring any component of the trainer appliance according to the invention to be dismounted and remounted for this purpose.

The above description has solely been given for illustrating the principle of the invention by way of example, numerous modifications and variations being allowed within the purview of the present invention.

## Claims

1. Apparatus for determining and controlling the free rotation of two arms (1, 2) about a pivot pin (3), particularly for use in an orthopedic (trainer) appliance for determining the minimum and maximum rotary positions of two limbs joined to one another by an articulation,
characterized in that
one (2) of said arms has disposed thereon a flat bar (4) of dimensions corresponding to those of said arms, said bar being provided with at least one hollow extension (5) along a longitudinal side thereof,
that the cylindrical hollow interior of said extension (5) contains a tension spring (7) having one of its ends fixed to the end (8) of the hollow extension, and its other end, to a plug (9) preferably of reduced-length cylindrical shape,
that said hollow interior also contains a hollow threaded member (11) disposed at a location opposite said plug (9), the latter being dimensioned so as to prevent it form entering the bore of said hollow threaded member (11), that a bushing (10) having internal threads in permanent engagement with said threaded member (11) is disposed at a fixed intermediate location of said extension (5) for rotation about the hollow interior thereof,
and that a central body (12) fixedly connected to said arm (2) is formed with an arcuate groove (13) extending around said pivot pin (3) and having disposed therein adjacent one of its ends an anchor pin (15) having attached thereto a cable, preferably a steel cable (16) extending in said arcuate groove (13) for a certain length and exiting therefrom to pass through said hollow threaded member (11) and to have its other end secured to said plug (9).

2. Apparatus according to claim 1, preferably made of carbon fiber or the like, characterized in that said flat bar (4) is provided with a second hollow extension (5b) disposed in symmetrical fashion along the opposite side of said bar (4), said second extension (5b) housing a mechanism mechanically and functionally identical to the construction described in claim 1, and including a corresponding flexible cable (16b) and associated anchor pin (15b).

3. Apparatus according to claim 1 and/or 2, characterized in that said springs (7, 7b) are disposed between the respective plugs (9, 9b) and the outer ends of the respective threaded members (11, 11b), said springs acting as compression springs.

4. Apparatus according to any of the preceding claims, characterized in that said flat bar (4) is eliminated and the functional components, namely, said hollow threaded member (11), said threaded bushing (10), said plug (9), said spring (7), said outer spring anchoring point (8) and said flexible cable (16), and if applicable, the corresponding components 11b, 10b, 9b, 7b, 8b and 16b, are accommodated within one of said arms (1, 2).

5. Apparatus according to any of the preceding claims, characterized in that said rotatable bushings (10,10b) are adapted to be locked in any position.

6. Apparatus according to any of the preceding claims, characterized in that said arms (1 and 2) are of significantly different lengths and widths.

7. Apparatus according to any of the preceding claims, characterized in that said groove (13) is of circular arc configuration extending about an arc of about 200^{o} with its center coaxial with said pivot pin (3).
